Europäisches Patentamt

**European Patent Office**

Office européen des brevets

(19)

(11) Publication number : **0 596 973 B1**

(12) # EUROPEAN PATENT SPECIFICATION

(45) Date of publication of patent specification :
**27.12.95 Bulletin 95/52**

(51) Int. Cl.⁶ : **G01N 27/12**

(21) Application number : **92916413.5**

(22) Date of filing : **29.07.92**

(86) International application number :
**PCT/GB92/01401**

(87) International publication number :
**WO 93/03355 18.02.93 Gazette 93/05**

(54) **DEVICE FOR SENSING VOLATILE MATERIALS**

(30) Priority : **29.07.91 GB 9116360**

(43) Date of publication of application :
**18.05.94 Bulletin 94/20**

(45) Publication of the grant of the patent :
**27.12.95 Bulletin 95/52**

(84) Designated Contracting States :
**AT BE CH DE DK ES FR GB GR IT LI LU MC NL SE**

(56) References cited :
**WO-A-86/01599**
**GB-A- 2 203 249**
**US-A- 4 415 876**
**US-A- 5 034 192**

(73) Proprietor : **NEOTRONICS LIMITED**
**Parsonage Road**
**Takeley**
**Bishop's Stortford Hertfordshire CM22 6PU (GB)**
Proprietor : **BASS BREWERS LIMITED**
**137 High Street**
**Burton-on-Trent, Staffordshire DE14 1JZ (GB)**

(72) Inventor : **GARDINER, Julian, W.**
**Department of Engineering**
**University of Warwick**
**Coventry CV4 7AL (GB)**
Inventor : **BARTLETT, Philip, N.**
**School of Chemistry**
**Claverton Down**
**Bath BA2 7AY (GB)**

(74) Representative : **Hedley, Nicholas James Matthew**
**Stephenson Harwood**
**One, St. Paul's Churchyard**
**London EC4M 8SH (GB)**

EP 0 596 973 B1

## Description

Technical Field

The present invention relates to a device for sensing volatile material in the gas phase, the device comprising: (a) a plurality of sensors each comprising a pair of spaced-apart electrical contacts and a semiconductor polymer extending between the contacts, wherein the polymer is such that its electrical resistance changes when exposed to volatile materials; and (b) means for measuring electrical resistance of the semiconductor polymer of each sensor or a parameter varying with said resistance or functionally related thereto

Background Art

Currently, there is no device commercially available for assessing the quality of a product based on its taste and aroma and this task is usually performed by tasting panels. However, tasting panels are expensive to convene, are not always able to detect the subtle changes in taste or aroma and it is not economic to monitor the quality of, for example, all batches of beer produced in a large brewery using a tasting panel. The same is true of other beverages and foodstuffs not only for human consumption but also for animal consumption.

It is known from International Application No.86/01599 (Persaud et al) and an article by Pelosi and Persaud ('Gas Sensors: Towards an Artificial Nose' NATO ASI Series, Vol F43, Sensors and Sensory Systems for Advanced Robots. Ed-P. Dario, Springer-Verlag Berlin Heidelberg 1988) to detect odours and gases by means of a device that is said to mimic the olfactory system.

The present invention is concerned with a device having an array of sensors each of which has a different response to a volatile material or a mixture of volatile materials, such as an odour, so that a "fingerprint" of the odour can be built up from the response given by the sensors in the array. Each sensor in the array is composed of a pair of contacts that are bridged by an organic semi-conductive polymer such as polypyrrole that may be grown electrochemically in the presence of a counter-ion between the two contacts. In each sensor of the array the resistance across the semi-conductive polymer is measured and because the polymers in the sensors in the array are different, their resistance changes in a different way on exposure to the volatile material(s). In this way, the fingerprint of the odour can be built up. For example, the polymers of certain sensors will interact with (possibly by absorption) low molecular weight materials more readily than the polymers of other sensors and accordingly the polymers that interact with the low molecular weight components of an odour will give a different response to those that do not interact with such low molecular weight components.

Each sensor can differ from other sensors in the array as a result of the different polymer used, e.g. one polymer could be polypyrrole and another could be poly(N-methylpyrrole). Alternatively, two (or more) sensors can utilise the same basic polymer (e.g. both could be polypyrrole) but their responses could differ because the polymer is grown in the presence of a different counter-ion. This difference arises because the polymer must be oxidised in order to be conducting and so, to balance the charge in the polymer, counter-ions are incorporated into the polymer, e.g. in a proportion of approximately one counter-ion to four monomer units. The nature of the counter-ion alters the resistive characteristics of the polymer when exposed to volatile material(s). Another factor affecting the resistivity characteristics of the polymer when exposed to volatile material(s) is the solvent used when growing the polymer.

The polymer used in the sensors can, for example, be chosen from: polypyrrole, poly N-methylpyrrole, poly 3-methylthiophene, polyaniline, poly-5-carboxyindole, poly-3-methyldiphenylamine, polybithiophene, polythiophene, poly-3-thiopheneacetic acid and polyfuran.

Typical counter-ions that can be used in growing the polymer of the sensors may be one or more of: tetrafluoroborate, alkyl sulphonates, tetramethylammonium chloride, chlorates and perchlorates.

Solvents used in polymer growth can be or include water, ethanol or acetonitrile.

Unfortunately, the polymers in the sensors tend to degrade rapidly, resulting in sensors having only a relatively short operational lifespan.

The present invention provides improvements in the above system.

WO-A-88/01599 describes a sensor for volatile material having a pair of spaced-apart contacts and a semiconductive polymer bridging the contacts. The spacing between the contacts is 200 $\mu$m.

GB-2203249 describes a sensor having a pair of contacts that are bridged by tin oxide.

US-5034192 describes a sensor comprising electrodes separated by a gap of less than 5 $\mu$m, but it measures the redox potential of the species being sensed and operates only in solutions.

Disclosure of the Invention

In accordance with the present invention, there is provided a device for sensing volatile materials as claimed in the accompanying claims.

The present invention also provides the use of such a device in the sensing of volatile materials in a gaseous atmosphere as set out in the accompanying claims.

We have found that providing a spacing between the contacts of 7 to 25 µm (as opposed to a spacing of about 100 µm in prior art sensors) the polymer does not degrade as rapidly and the life-span of the sensors in the device of the present invention is extended. A further advantage of the present invention is that the response time is shortened as compared to the prior art.

The present invention also provides a method of making the device, which method comprises growing the polymer of each sensor electrochemically by placing the contacts of each sensor in a solution containing a monomer precursor of the polymer and a counter-ion and maintaining a substantially constant potential or a cyclically scanned potential between the contacts and a reference electrode by means of an electrical circuit e.g. a potentiostatic circuit, during polymerisation of the monomer, which circuit causes a current to flow between the contacts and a counter electrode.

The reference electrode may be a standard calomel electrode or an Ag/AgCl electrode. A potential of approximately 1 volt should be applied between the two contacts (typical value of 1V) and the counter electrode in order to grow the polymer. The advantage of this aspect of the present invention is that the growth of the polymer can be achieved in a more reproducible manner than is the case when a potential is simply applied between the contacts and a counter electrode and so the sensor thus formed will give more reproducible results. The polymer is preferably (but not necessarily) grown on a substrate, which is most preferably an alumina substrate or a fused quartz substrate; other suitable substrate materials may be envisaged by persons skilled in the art.

It is possible to regulate the amount of polymer grown by terminating the polymer growth after a predetermined period of time or when a predetermined amount of current has passed. Alternatively, a small potential difference can be maintained or periodically applid between the two contacts of the sensor or a small amplitude a.c. signal can be applied across the two contacts of the sensor to monitor the growth of the semi-conductor polymer using a bipotentiostatic circuit; polymer growth may be terminated when the resistance of the semi-conductor polymer during its growth reaches a threshold value.

After the polymer has been grown, a potential difference may be maintained between the contacts and the counter electrode which is lower than that at which substantial polymer growth occurs. We have found that maintaining the polymer at a potential lower than the growth potential allows equilibration of the counter-ion within the polymer. It is also possible, according to this aspect of the present invention, to change the counter-ion in the polymer by removing the polymer from the solution in which it was grown and dipping it into a further solution containing a different counter-ion and maintaining the polymer at the said potential lower than the polymer growth potential until the substitute counter-ion has replaced the original counter-ions in the polymer.

The contacts and the polymer are preferably supported on an alumina substrate which provides excellent adhesion between the polymer and the substrate.

In accordance with a preferred aspect, the sensors may each be made by depositing a metal (preferably gold) onto a substrate (preferably onto an alumina substrate) to form the said contacts, depositing a photoresist mask over the electrodes, which mask exposes a locus corresponding to the desired position of the polymer (the locus of the polymer being a limited area between the contacts together with parts overlying the two contacts that enable electrical contact to be established between the contacts and the polymer), dipping the substrate into a solution of a monomer precursor of the polymer and applying a potential difference between the contacts and a reference electrode to cause the monomer precursor to polymerise between the contacts in the said locus.

The photoresist, which will be of a low bake type and electrochemically inert, preferably covers the whole of the contacts except for the locus of the polymer and a further area allowing the contacts to be connected to external detecting and controlling circuit. The photoresist can remain in place to protect the contacts in use and thus improve the ruggedness of the sensor, which prevents damage to the contacts and in particular prevents the contacts from exfoliating from the substrate.

Brief Description of Drawings

The present invention will now be described in further detail with reference to the accompanying drawings, in which:

Figure 1 shows a mask for use in making a sensor in accordance with the present invention;

Figure 2 shows a partly made sensor in accordance with the present invention;

Figure 3 shows a further mask for use in making a sensor in accordance with the present invention;

Figure 4 shows a partly made sensor in accordance with the present invention and

Figure 5 shows a third mask for use in making a sensor in accordance with the present invention.

Figure 6 is a schematic drawing of three sensors for use in the device of the present invention.

Best Modes for Carrying out the Invention

A 0.5 inch (1.25 cm) square tile made of alumina is used as a substrate for a sensor and the sensor was built up on it as follows:

(1) A layer of gold 500 Angstrom thick was vapour-deposited over one side of the square tile.

(2) Dust particles are removed from the gold-coated substrate by washing it with xylene.

(3) A photoresist (Shipley positive TF-16) was applied to the substrate by spinning for 15 seconds.

(4) The substrate covered by the resist was soft-baked in an oven for 20 minutes at 90°C.

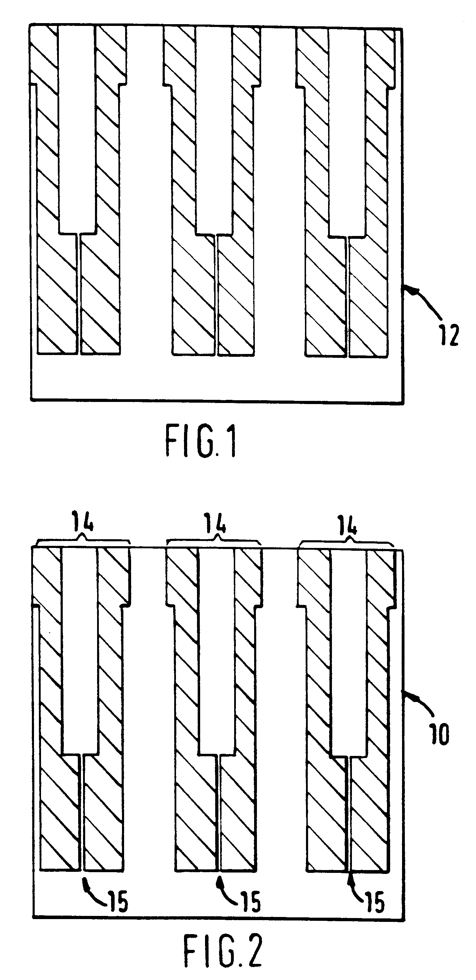

(5) A mask 12 (shown in Figure 1 where the shaded area are translucent) was placed against the resist and the resist was exposed to ultra-violet light through the mask for 4 minutes.

(6) The unexposed area of the photoresist was removed using Shipley MF-319 developer for 1 minute.

(7) The gold-coated substrate was washed in distilled water and hard-baked in an oven for 1 hour at 115°C to render the resist insoluble to an etchant used in step (8) below.

(8) At the area where the resist was removed, the gold was etched using a gold-etchant; the etching time was 1 minute.

(9) The remaining photoresist was removed using acetone and the substrate was washed in distilled water. Thus, referring to Figure 2, the substrate 10 bears a gold image of mask 12 and this image forms three pairs of contacts 14 each pair of contacts being part of a different sensor. The contacts of each pair are separated by a gap 15 to 10 μm in width.

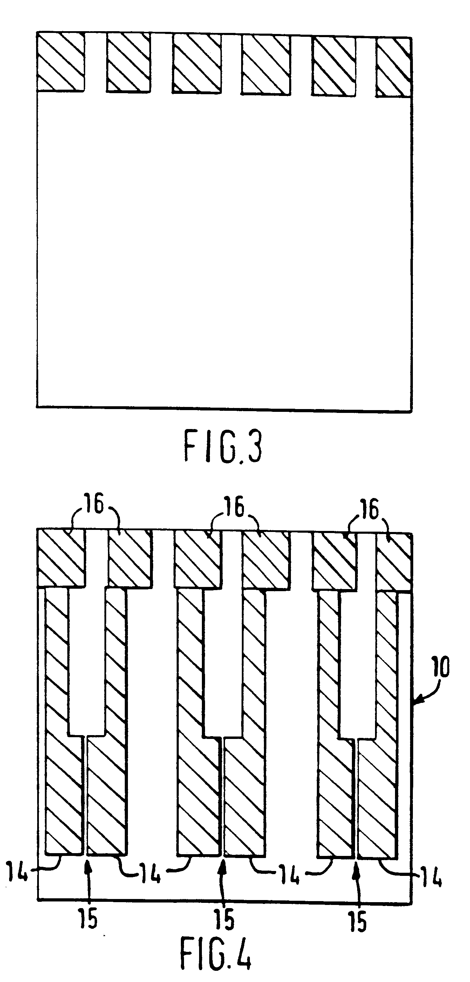

(10) A brass mask (shown in Figure 3 where the holes cut in the mask are shaded) was placed against the substrate and chromium was vapour-deposited on top of the gold contacts to form contact pads for forming electrical connections to a monitoring circuit. The substrate, contacts and contact pads 16 are shown in Figure 4. Alternatively, the contact pads may be deposited after the polymer growth.

(11) Xylene was spun over the surface of the substrate to remove any dust particles.

(12) A Shipley 1813 photoresist was spun over the surface of the substrate for 15 seconds.

(13) The substrate bearing the contacts and the contact pads was soft-baked in an oven for 5 minutes at 90°C.

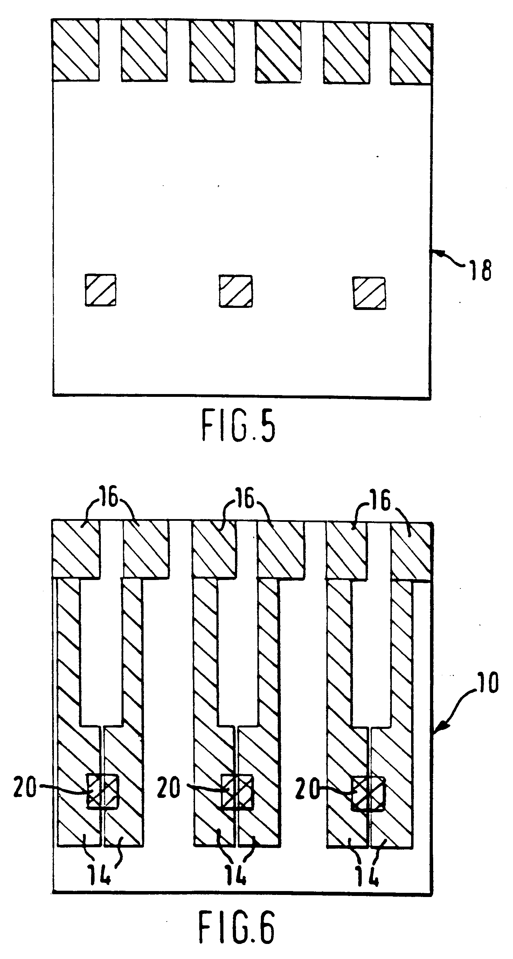

(14) The photoresist was exposed to ultra-violet light through a mask 18 (shown in Figure 5, the shaded areas being opaque) for 12 minutes.

(15) The photoresist was developed using equal parts of Shipley 351 developer diluted with water; the developing time was 1 minute.

(16) The substrate was then washed in distilled water and hard-baked at 180°C for 1 hour; this temperature caused the photoresist to become insoluble in the various solutions used in the electrochemical growth of the polymer.

(17) The masked substrate was cleaned by cycling in 1 molar sulphuric acid between -0.3 and 1.8 volts at 100 millivolts/second for 3 cycles. After cleaning, the substrate was stored in pure water until required.

(18) The masked substrate was placed in a monomer solution (details of the composition of which are given below) and both contacts of the device are connected to a potentiostatic circuit that maintains the potential of the contacts substantially with respect to a calomel reference electrode; a counter electrode is also provided.

(19) The potential between the contacts and a reference electrode is stepped or cycled to a predetermined working voltage to begin growth. After the desired time, the potential is stepped back to 0 V. If after viewing the device under the microscope or by measuring the resistance of the polymer it becomes apparent that the gap between the contacts has not been crossed, then the electrode can be returned to the working solution and further growth performed.

(20) The polymer is maintained in the monomer solution at a potential less than the growth potential for a sufficient time to ensure the concentration of counter-ions in the polymer has equilibrated.

(21) The device is washed in the solvent used to make the growth solution. This removes any surface debris and prevents any drying marks due to electrolyte deposition.

(22) The conductivity of the polymer was then tested.

Figure 6 shows the tile bearing three sensors. For ease of illustration, the top photoresist, which covers the whole of the tile-10 except for the contact pads 16 and the areas 20 in which the polymer is deposited, has been omitted. In use, the resistivity of the polymer 20 between each pair of contacts 12 is measured by passing a current through the polymer by means of the contacts. The resistivity of each sensor is analysed using known pattern-recognition techniques to give an indication of the volatile material(s) present in a sample to which the sensors are exposed.

The following solutions of monomers have been used successfully to grow polymers between the contacts:

1)

| | |
|---|---|
| Monomer concentration | : 0.1 M pyrrole |
| Counter-ion concentration | : 0.1 M tetraethylammonium tetrafluoroborate |
| Solvent | : Water |
| Growth method | : Potential step 0 to 0.8 V |
| Growth time | : 3 minutes |

The polypyrrole produced was smooth, strong and black.

2)

| | |
|---|---|
| Monomer concentration | : 0.1 M pyrrole |
| Counter-ion concentration | : 0.1 M lithium triflate |
| Solvent | : Water |
| Growth method | : Potential step 0-0.9 V |
| Growth time | : 4 minutes |

The polymer had to be held at a potential below the growth potential (0.9V) to allow equilibration of the counter-ions in the polymer otherwise the polymer was non-conductive.

3)

| | |
|---|---|
| Monomer concentration | : 0.1 M N-methylpyrrole |
| Counter-ion concentration | : 0.1 M sodium salt of dodecylbenzene sulphonic acid |
| Solvent | : Water |
| Growth method | : Potential step 0 - (07.-0.9) V |
| Growth time | : at least 16 minutes |

4)

| | |
|---|---|
| Monomer concentration | : 0.1 M 3-methylthiophene |
| Counter-ion concentration | : 0.1 M tetraethylammonium tetrafluoroborate |
| Solvent | : Acetonitrile |
| Growth method | : Potential step 0 - 1.65 V |
| Growth time | : <3 minutes |

5)

| | |
|---|---|
| Monomer concentration | : 0.1 M pyrrole |
| Counter-ion concentration | : 0.1 M tetraethylammmonium toluene-sulphonate |
| Solvent | : Water |
| Growth method | : Potential step 0 - 0.8 V |
| Growth time | : 3 minutes |

6)

| | |
|---|---|
| Monomer concentration | : 0.1 M 3-methyldiphenylamine |
| Counter-ion concentration | : 0.1 M $LiClO_4$ |
| Solvent | : Acetonitrile |
| Growth method | : Potential step 0 - 1.0 V |
| Growth time | : 2 minutes |

7)

| | |
|---|---|
| Monomer concentration | : 0.05 M 2,2'-bithiophene |
| Counter-ion concentration | : 0.1 M tetraethylammonium tetrafluoroborate |
| Solvent | : Acetonitrile |
| Growth method | : Potential step 0 - 1.65 V |
| Growth time | : < 3 minutes |

8)

| | |
|---|---|
| Monomer concentration | : 0.1 M pyrrole |
| Counter-ion concentration | : 0.1 M octanesulphonic acid |
| Solvent | : Water |
| Growth method | : Potential step 0 - 0.8 V |
| Growth time | : 2 minutes |

The films are dense, black and smooth with good adhesion. DC resistance is typically 50 $\Omega$.

9)

5

| Monomer concentration | : 0.1 M pyrrole |
| Counter-ion concentration | : 0.1 M hexanesulphonic acid |
| Solvent | : Water |
| Growth method | : Potential step 0 - 0.8 V |
| Growth time | : 2 minutes |

The films are dense, black and smooth with good adhesion. DC resistance is typically 50 $\Omega$.

10)

| Monomer concentration | : 0.1 M pyrrole |
| Counter-ion concentration | : 0.001 M anthraquinone-2-sulphonic acid |
| Solvent | : Water |
| Growth method | : Potential step 0 - 1.15 V |
| Growth time | : 2 minutes |

11)

| Monomer concentration | : 0.1 M pyrrole |
| Counter-ion concentration | : 0.1 M butanesulphonic acid |
| Solvent | : Water |
| Growth method | : Potential step 0 - 0.85 V |
| Growth time | : 2 minutes |

12)

| Monomer concentration | : 0.1 M pyrrole |
| Counter-ion concentration | : 0.1 M pentanesulphonic acid |
| Solvent | : Water |
| Growth method | : Potential step 0 - 0.85 V |
| Growth time | : 2 minutes |

13)

| Monomer concentration | : 0.1 M pyrrole |
| Counter-ion concentration | : 0.1 M heptanesulphonic acid |
| Solvent | : Water |
| Growth method | : Potential step 0 - 0.85 V |
| Growth time | : 2 minutes |

14)

| Monomer concentration | : 0.1 M pyrrole |
| Counter-ion concentration | : 0.1 M decanesulphonic acid |
| Solvent | : Water |
| Growth method | : Potential step 0 - 0.85 V |
| Growth time | : 2 minutes |

15)

| Monomer concentration | : 0.44 M aniline |
| Counter-ion concentration | : 0.5 M NaHSO$_4$,Ph 1 |
| Solvent | : Water |
| Growth method | : Potential step 0 - 0.9 V |
| Growth time | : 2 minutes |

16)

| Monomer concentration | : 0.1 M pyrrole |
| Counter-ion concentration | : 0.1 M $\rho$-toluene sulphonic acid |
| Solvent | : Ethanol |
| Growth method | : Potential step 0 - 1.2 V |
| Growth time | : 2 minutes |

17)

| Monomer concentration | : 0.1 M pyrrole |
| Counter-ion concentration | : 0.1 M tetraethylammonium tetrafluoroborate |
| Solvent | : Acetonitrile |
| Growth method | : Potential step 0 - 1.1 V |
| Growth time | : 2 minutes |

Sensors having different semi-conductive polymers and/or having polymers grown in the presence of different counter-ions or in the presence of different solvents can be combined into an array. When the array of sensors is exposed to a volatile vapour, the change in the conductivity of the polymer of each sensor is measured and the response given by the sensors in the array will depend on the nature of the volatile materials, different volatile materials generally giving different response patterns. These different response patterns can be recognised using known pattern recognition techniques to give a response giving an indication of the nature of the volatile materials in the vapour.

An array of eleven sensors that we have used is as follows:

| Sensor No. | Type of polymer/counter-ion/solvent |
|---|---|
| 0 | Polypyrrole/butanesulphonate/water |
| 1 | Polypyrrole/butanesulphonate/water |
| 2 | Polypyrrole/toluenesulphonate/ethanol |
| 3 | Polypyrrole/toluenesulphonate/ethanol |
| 4 | Polypyrrole/tetraethyleneammonium toluene-sulphonate/water |
| 5 | Polypyrrole/tetraethyleneammonium toluene-sulphonate/water |
| 6 | Poly-3-methylthiophene/tetraethyleneammonium-tetrafluoroborate/acetonitrile |
| 7 | Polypyrrole/propanesulphonate/water |
| 8 | Polypyrrole/propanesulphonate/water |
| 9 | Polyaniline/bisulphate/water |
| 10 | Polyaniline/bisulphate/water |

This array has been able to distinguish between different brands of lager beer and between two samples of the same beer that had been stored differently.

**Claims**

1. A device for sensing volatile material in the gas phase, the device comprising:
   (a) a plurality of sensors each comprising a pair of electrical contacts (14) spaced apart by a gap and supported on a substrate and a semi-conductive polymer (20) located in a predetermined limited area and extending across the gap between the contacts, wherein the polymer is such that its electrical resistance can change when exposed to volatile materials; and
   (b) means for measuring the electrical resistance of the semi-conductive polymer of each sensor or a parameter varying with said resistance or functionally related thereto, wherein the width of the gap between the contacts in each sensor across which the polymer extends is 7 to 25 $\mu$m, preferably 7 to 13 $\mu$m, more preferably 9 to 11 $\mu$m and most preferably about 10 $\mu$m, and wherein, except in the said gap, the contacts extend under the whole of the said limited area.

2. A device as claimed in claim 1, wherein the substrate (10) is an alumina or fused quartz substrate.

3. A device as claimed in claim 1 or claim 2, which includes a photoresist which covers each sensor except for the polymer.

4. A device as claimed in any one of claims 1 to 3, wherein the width of the said polymer on each side of the said gap is greater than the width of the gap itself.

5. A method of making a device according to any one of claims 1 to 4, which comprises growing the polymer (20) of each sensor electrochemically by placing the contacts (14) of each sensor in a solution containing a monomer precursor of the polymer and a counter-ion and maintaining a substantially constant potential or a cyclically scanned potential between the contacts and, a reference electrode by means of an electrical circuit during polymerisation of the monomer, which circuit causes a current to flow between the contacts and a counter electrode thereby causing the monomer to polymerise between contacts.

6. A method as claimed in claim 5, wherein a constant, alternating or periodic potential difference is imposed between the contacts (14) and wherein the polymer growth is terminated when the resistance of the polymer during its growth reaches a threshold value.

7. A method as claimed in claim 5 or claim 6, wherein, after the polymer has been grown, a potential difference is maintained between the contacts and the counter electrode, which potential is lower than that at which substantial polymer growth occurs, thereby equilibrating the concentration of the counter-ion within the polymer.

8. A method as claimed in any one of claims 5 to 7, which further comprises, prior to growing the polymer, depositing the contacts (14) of each sensor onto a substrate, depositing a photoresist mask (18) over the contacts, which mask leaves exposed a limited area between the contacts corresponding to the desired position of the polymer (20) and wherein the polymer is grown in the said limited area.

9. A method as claimed in any one of claims 5 to 8, wherein the polymer (20) of at least one of the sensors is grown from a monomer selected from the following:

a)

| | |
|---|---|
| Monomer concentration | : 0.1 M pyrrole |
| Counter-ion concentration | : 0.1 M tetraethylammonium tetrafluoroborate |
| Solvent | : Water |

b)

| | |
|---|---|
| Monomer concentration | : 0.1 M pyrrole |
| Counter-ion concentration | : 0.1 M lithium triflate |
| Solvent | : Water |

c)

| | |
|---|---|
| Monomer concentration | : 0.1 M N-methylpyrrole |
| Counter-ion concentration | : 0.1 M sodium salt of dodecylbenzene sulphonic acid |
| Solvent | : Water |

d)

| | |
|---|---|
| Monomer concentration | : 0.1 M 3-methylthiophene |
| Counter-ion concentration | : 0.1 M tetraethylammonium tetrafluoroborate |
| Solvent | : Acetonitrile |

e)

| | |
|---|---|
| Monomer concentration | : 0.1 M pyrrole |
| Counter-ion concentration | : 0.1 M tetraethylammonium toluene-sulphonate |
| Solvent | : Water |

f)

| | |
|---|---|
| Monomer concentration | : 0.1 M 3-methyldiphenylamine |
| Counter-ion concentration | : 0.1 M $LiClO_4$ |
| Solvent | : Acetonitrile |

g)

| | |
|---|---|
| Monomer concentration | : 0.05 M 2,2'-bithiophene |
| Counter-ion concentration | : 0.1 M tetraethylammonium tetrafluoroborate |
| Solvent | : Acetonitrile |

h)

| | |
|---|---|
| Monomer concentration | : 0.1 M pyrrole |
| Counter-ion concentration | : 0.1 M octanesulphonic acid |
| Solvent | : Water |

i)

| | |
|---|---|
| Monomer concentration | : 0.1 M pyrrole |
| Counter-ion concentration | : 0.1 M hexanesulphonic acid |
| Solvent | : Water |

j)

| | |
|---|---|
| Monomer concentration | : 0.1 M pyrrole |
| Counter-ion concentration | : 0.001 M anthraquinone-2-sulphonic acid |
| Solvent | : Water |

k)

| | |
|---|---|
| Monomer concentration | : 0.1 M pyrrole |
| Counter-ion concentration | : 0.1 M butanesulphonic acid |
| Solvent | : Water |

l)

| | |
|---|---|
| Monomer concentration | : 0.1 M pyrrole |
| Counter-ion concentration | : 0.1 M pentanesulphonic acid |
| Solvent | : Water |

m)

| | |
|---|---|
| Monomer concentration | : 0.1 M pyrrole |
| Counter-ion concentration | : 0.1 M heptanesulphonic acid |
| Solvent | : Water |

n)

| | |
|---|---|
| Monomer concentration | : 0.1 M pyrrole |
| Counter-ion concentration | : 0.1 M decanesulphonic acid |
| Solvent | : Water |

o)

| | |
|---|---|
| Monomer concentration | : 0.44 M aniline |
| Counter-ion concentration | : 0.5 M $NaHSO_4$, Ph 1 |
| Solvent | : Water |

p)

| | |
|---|---|
| Monomer concentration | : 0.1 M pyrrole |
| Counter-ion concentration | : 0.1 M $\rho$-toluene sulphonic acid |
| Solvent | : Ethanol |

q)

| | |
|---|---|
| Monomer concentration | : 0.1 M pyrrole |
| Counter-ion concentration | : 0.1 M tetraethylammonium tetrafluoroborate |
| Solvent | : Acetonitrile |

10. Use of a device of any one of claims 1 to 3 for sensing volatile materials in the gas phase whereby the sensors are exposed to a gaseous atmosphere containing such volatile materials and the resistance of the polymer material (20) of each sensor or a parameter varying with the said resistance or functionally related thereto is measured to provide a response for the said volatile materials in the atmosphere.

**Patentansprüche**

1. Vorrichtung zur Wahrnehmung von flüchtigem Stoff in der Gasphase, mit:
   (a) einer Vielzahl von Sensoren, die jeder ein Paar von elektrischen Kontakten (14) aufweisen, die durch einen Spalt zueinander beabstandet sind und auf dem Substrat getragen werden, und einem halbleitenden Polymer (20), das in einem vorbestimmten begrenzten Bereich angeordnet ist und sich über den Spalt zwischen den Kontakten erstreckt, wobei das Polymer derart ist, daß sich sein elektrischer Widerstand ändern kann, wenn es flüchtigen Stoffen ausgesetzt wird; und
   (b) Mitteln zum Messen des elektrischen Widerstandes des halbleitenden Polymers jedes Sensors oder eines Parameters, der sich mit dem besagten Widerstand ändert oder mit diesem funktional verbunden ist, wobei die Breite des Spaltes zwischen den Kontakten bei jedem Sensor, über den sich das Polymer erstreckt, zwischen 7 und 25 μm ist, vorzugsweise 7 bis 13 μm, noch bevorzugterweise 9 bis 11 μm und am bevorzugtesten etwa 10 μm, und wobei, außer in dem genannten Spalt, sich die Kontakte unter der Gesamtheit des genannten begrenzten Bereiches erstreckt.

2. Vorrichtung gemäß Anspruch 1,
   wobei das Substrat (10) ein Aluminiumoxid- oder geschmolzenes Quarzsubstrat ist.

3. Vorrichtung gemäß Anspruch 1 oder 2,
   die einen Fotoabdecklack aufweist, der jeden Sensor mit Ausnahme des Polymers abdeckt.

4. Vorrichtung gemäß einem der Ansprüche 1 bis 3,
bei dem die Breite des besagten Polymers auf jeder Seite des besagten Spaltes größer ist als die Breite des Spaltes selbst.

5. Verfahren zur Herstellung einer Vorrichtung gemäß einem der Ansprüche 1 bis 4,
das umfaßt, das Polymer (20) jedes Sensors elektrochemisch wachsen zu lassen durch Einbringen der Kontakte (14) jedes Sensors in eine Lösung, die einen monomeren Zwischenstoff des Polymers und ein Gegenion enthält sowie Beibehaltung eines im wesentlichen konstanten Potentials oder eines zyklisch abgetasteten Potentials zwischen den Kontakten und einer Referenzelektrode mittels eines elektrischen Schaltkreises während der Polymerisation des Monomers, wobei der Schaltkreis einen Strom veranlaßt, zwischen den Kontakten und der Gegenelektrode zu fließen, wodurch der Monomer veranlaßt wird, zwischen den Kontakten zu polymerisieren.

6. Verfahren gemäß Anspruch 5,
wobei eine konstante, alternierende oder periodische Potentialdifferenz zwischen den Kontakten (14) aufgebracht wird, und wobei das Polymerwachstum beendet wird, wenn der Widerstand des Polymers während des Wachsens einen Schwellenwert erreicht.

7. Verfahren gemäß Anspruch 5 oder 6,
wobei ein Potentialunterschied zwischen den Kontakten und der Gegenelektrode beibehalten wird, nachdem das Polymer wachsen gelassen wurde, wobei das Potential niedriger ist als das, bei dem das Polymerwachstum im wesentlichen auftritt, wodurch die Konzentration des Gegenions innerhalb des Polymers ausgeglichen wird.

8. Verfahren gemäß einem der Ansprüche 5 bis 7,
das weiterhin umfaßt, vor dem Wachsenlassen des Polymers die Kontakte (14) jedes Sensors auf einem Substrat abzulegen, eine Maske (18) aus Fotoabdecklack über die Kontakte zu legen, die einen begrenzten Bereich zwischen den Kontakten freiläßt, der der gewünschten Position des Polymers (20) entspricht und bei dem das Polymer in dem genannten begrenzten Bereich wachsen gelassen wird.

9. Verfahren gemäß einem der Ansprüche 5 bis 8,
bei dem das Polymer (20) von wenigstens einem der Sensoren aus einem Monomer wachsen gelassen wird, das aus den folgenden ausgewählt ist:

   a)

| | |
|---|---|
| Monomerkonzentration: | 0,1 M Pyrrol |
| Gegenionkonzentration: | 0,1 M Tetraethylammoniumtetrafluorborat |
| Lösungsmittel: | Wasser |

   b)

| | |
|---|---|
| Monomerkonzentration: | 0,1 M Pyrrol |
| Gegenionkonzentration: | 0,1 M Lithiumtriflat |
| Lösungsmittel: | Wasser |

   c)

| | |
|---|---|
| Monomerkonzentration: | 0,1 M N-Methylpyrrol |
| Gegenionkonzentration: | 0,1 M Natriumsalz von Dodecylbenzolsulfonsäure |
| Lösungsmittel: | Wasser |

   d)

| | |
|---|---|
| Monomerkonzentration: | 0,1 M 3-Mehtylthiophen |
| Gegenionkonzentration: | 0,1 M Tetraethylammoniumtetrafluorborat |
| Lösungsmittel: | Acetonitril |

   e)

| | |
|---|---|
| Monomerkonzentration: | 0,1 M Pyrrol |
| Gegenionkonzentration: | 0,1 M Tetraethylammoniumtoluol-Sulfonat |
| Lösungsmittel: | Wasser |

   f)

| | |
|---|---|
| Monomerkonzentration: | 0,1 M 3-Methyldiphenylamin |
| Gegenionkonzentration: | 0,1 M $LiClO_4$ |
| Lösungsmittel: | Acetonitril |

   g)

| | |
|---|---|
| Monomerkonzentration: | 0,05 M 2,2'-Bithiophen |
| Gegenionkonzentration: | 0,1 M Tetraethylammoniumtetrafluorborat |
| Lösungsmittel: | Acetonitril |

h)

| | |
|---|---|
| Monomerkonzentration: | 0,1 M Pyrrol |
| Gegenionkonzentration: | 0,1 M Octansulfonsäure |
| Lösungsmittel: | Wasser |

i)

| | |
|---|---|
| Monomerkonzentration: | 0,1 M Pyrrol |
| Gegenionkonzentration: | 0,1 M Hexansulfonsäure |
| Lösungsmittel: | Wasser |

j)

| | |
|---|---|
| Monomerkonzentration: | 0,1 M Pyrrol |
| Gegenionkonzentration: | 0,001 M Anthraquinon-2-Sulfonsäure |
| Lösungsmittel: | Wasser |

k)

| | |
|---|---|
| Monomerkonzentration: | 0,1 M Pyrrol |
| Gegenionkonzentration: | 0,1 M Butansulfonsäure |
| Lösungsmittel: | Wasser |

l)

| | |
|---|---|
| Monomerkonzentration: | 0,1 M Pyrrol |
| Gegenionkonzentration: | 0,1 M Pentansulfonsäure |
| Lösungsmittel: | Wasser |

m)

| | |
|---|---|
| Monomerkonzentration: | 0,1 M Pyrrol |
| Gegenionkonzentration: | 0,1 M Heptansulfonsäure |
| Lösungsmittel: | Wasser |

n)

| | |
|---|---|
| Monomerkonzentration: | 0,1 M Pyrrol |
| Gegenionkonzentration: | 0,1 M Decansulfonsäure |
| Lösungsmittel: | Wasser |

o)

| | |
|---|---|
| Monomerkonzentration: | 0,44 M Anilin |
| Gegenionkonzentration: | 0,5 M $NaHSO_4$, Ph 1 |
| Lösungsmittel: | Wasser |

p)

| | |
|---|---|
| Monomerkonzentration: | 0,1 M Pyrrol |
| Gegenionkonzentration: | 0,1 M $p$-Toluolsulfonsäure |
| Lösungsmittel: | Ethanol |

q)

| | |
|---|---|
| Monomerkonzentration: | 0,1 M Pyrrol |
| Gegenionkonzentration: | 0,1 M Tetraehtylammoniumtetrafluorborat |
| Lösungsmittel: | Acetonitril |

10. Gebrauch einer Vorrichtung gemäß einem der Ansprüche 1 bis 3 zur Feststellung von flüchtigen Stoffen in der Gasphase, wobei die Sensoren einer gasförmigen Atmosphäre ausgesetzt sind, die solche flüchtigen Stoffe enthält und der Widerstand des polymeren Materials (20) von jedem Sensor oder ein Parameter, der sich mit dem genannten Widerstand verändert oder funktionell mit diesem verbunden ist, gemessen wird, um eine Reaktion für den genannten flüchtigen Stoff in der Atmosphäre zu erhalten.

## Revendications

1. Dispositif pour détecter des matériaux volatils en phase gazeuse, le dispositif comprenant :

(a) une pluralité de détecteurs dont chacun comprend une paire de contacts électriques (14) séparés par un espace et supportés sur un substrat, et un polymère semi-conducteur (20) situé dans une région limitée prédéterminée et s'étendant d'un côté à l'autre de l'espace entre les contacts, le polymère étant tel que sa résistance électrique peut changer lorsqu'il est exposé au matériau volatil ; et

11

(b) un moyen de mesure de la résistance électrique du polymère semi-conducteur de chaque détecteur, ou d'un paramètre variant avec ladite résistance, ou en relation fonctionnelle avec celle-ci, dispositif dans lequel la largeur de l'espace entre les contacts de chaque détecteur sur lequel s'étend le polymère va de 7 à 25 µm, de préférence de 7 à 13 µm, mieux, de 9 à 11 µm, et encore mieux, est d'environ 10 µm, et dans lequel, excepté dans ledit espace, les contacts s'étendent sous toute ladite région limitée.

2. Dispositif selon la revendication 1, dans lequel le substrat (10) est un substrat en alumine ou en quartz fondu.

3. Dispositif selon la revendication 1 ou 2 qui comprend une photorésistance recouvrant chaque détecteur à l'exception du polymère.

4. Dispositif selon l'une quelconque des revendications 1 à 3, dans lequel la largeur dudit polymère de chaque côté dudit espace est supérieure à la largeur de l'espace lui-même.

5. Procédé de fabrication d'un dispositif selon l'une quelconque des revendications 1 à 4, qui consiste à faire croître le polymère (20) de chaque détecteur électrochimiquement en plaçant les contacts (14) de chaque détecteur dans une solution contenant un précurseur monomère dudit polymère et un contre-ion, et à maintenir un potentiel sensiblement constant ou un potentiel balayé cycliquement entre les contacts et une électrode de référence au moyen d'un circuit électrique pendant la polymérisation du monomère, lequel circuit provoque le passage d'un courant entre les contacts et une contre-électrode avec pour résultat que le monomère se polymérise entre les contacts.

6. Procédé selon la revendication 5, dans lequel une différence de potentiel constante, alternative ou périodique, est imposée entre les contacts (14) et dans lequel la croissance du polymère est terminée lorsque la résistance du polymère au cours de sa croissance atteint une valeur seuil.

7. Procédé selon la revendication 5 ou 6, dans lequel, après que le polymère ait crû, une différence de potentiel est maintenue entre les contacts et la contre-électrode, lequel potentiel est inférieur à celui auquel se produit une croissance sensible du polymère, équilibrant ainsi la concentration du contre-ion dans le polymère.

8. Procédé selon l'une quelconque des revendications 5 à 7, qui comprend en outre, avant la croissance du polymère, le dépôt des contacts (14) de chaque détecteur sur un substrat, le dépôt d'un masque photorésistant (18) sur les contacts, lequel masque laisse exposée une région limitée entre les contacts correspondant à la position voulue pour le polymère (20) et dans lequel le polymère croît dans ladite région limitée.

9. Procédé selon l'une quelconque des revendications 5 à 8, dans lequel le polymère (20) de l'un au moins des détecteurs croît à partir d'un monomère choisi parmi les suivants :

a)

| | |
|---|---|
| Concentration en monomère | : 0,1 M pyrrole |
| Concentration en contre-ion | : 0,1 M tétrafluoroborate de tétraéthylammonium |
| Solvant | : Eau |

b)

| | |
|---|---|
| Concentration en monomère | : 0,1 M pyrrole |
| Concentration en contre-ion | : 0,1 M triflate de lithium |
| Solvant | : Eau |

c)

| | |
|---|---|
| Concentration en monomère | : 0,1 M N-méthylpyrrole |
| Concentration en contre-ion | : 0,1 M sel de sodium de l'acide dodécylbenzène sulfonique |
| Solvant | : Eau |

d)

| | |
|---|---|
| Concentration en monomère | : 0,1 M 3-méthylthiophène |
| Concentration en contre-ion | : 0,1 M tétrafluoroborate de tétraéthylammonium |
| Solvant | : Acétonitrile |

e)

| | |
|---|---|
| Concentration en monomère | : 0,1 M pyrrole |
| Concentration en contre-ion | : 0,1 M toluène-sulfonate de tétraéthylammonium |

Solvant : Eau

f)
Concentration en monomère : 0,1 M 3-méthyldiphénylamine
Concentration en contre-ion : 0,1 M LiClO4
Solvant : Acétonitrile

g)
Concentration en monomère : 0,05 M 2,2'-bithiophène
Concentration en contre-ion : 0,1 M tétrafluoroborate de tétraéthylammonium
Solvant : Acétonitrile

h)
Concentration en monomère : 0,1 M pyrrole
Concentration en contre-ion : 0,1 M acide octane-sulfonique
Solvant : Eau

i)
Concentration en monomère : 0,1 M pyrrole
Concentration en contre-ion : 0,1 M acide hexane-sulfonique
Solvant : Eau

j)
Concentration en monomère : 0,1 M pyrrole
Concentration en contre-ion : 0,001 M acide anthraquinone-2-sulfonique
Solvant : Eau

k)
Concentration en monomère : 0,1 M pyrrole
Concentration en contre-ion : 0,1 M acide butane-sulfonique
Solvant : Eau

l)
Concentration en monomère : 0,1 M pyrrole
Concentration en contre-ion : 0,1 M acide pentane-sulfonique
Solvant : Eau

m)
Concentration en monomère : 0,1 M pyrrole
Concentration en contre-ion : 0,1 M acide heptane-sulfonique
Solvant : Eau

n)
Concentration en monomère : 0,1 M pyrrole
Concentration en contre-ion : 0,1 M acide décane-sulfonique
Solvant : Eau

o)
Concentration en monomère : 0,44 M aniline
Concentration en contre-ion : 0,5 M $NaHSO_4$ Ph 1
Solvant : Eau

p)
Concentration en monomère : 0,1 M pyrrole
Concentration en contre-ion : 0,1 M acide p-toluène-sulfonique
Solvant : Ethanol

q)
Concentration en monomère : 0,1 M pyrrole
Concentration en contre-ion : 0,1 M tétrafluoroborate de tétraéthylammonium
Solvant : Acétonitrile

10. Utilisation d'un dispositif selon l'une quelconque des revendications 1 à 3 pour détecter des matériaux volatils en phase gazeuse, dans laquelle les détecteurs sont exposés à une atmosphère gazeuse contenant de tels matériaux volatils et la résistance du matériau polymère (20) de chaque détecteur, ou un paramètre variant avec ladite résistance, ou en relation fonctionnelle avec celle-ci, est mesuré pour donner une réponse traduisant la présence desdits matériaux volatils dans l'atmosphère.

13

FIG.1

FIG.2

FIG.3

FIG.4

FIG.5

16      16      16

20    20    20

14    14    14

10

FIG.6